# EUROPÄISCHE PATENTSCHRIFT

(11) **EP 1 651 117 B1**
(45) Veröffentlichungstag und Bekanntmachung des Hinweises auf die Patenterteilung: **24.01.2007**
(21) Anmeldenummer: 04801927.7
(22) Anmeldetag: 18.08.2004
(51) Int. Cl.: A61B 17/00

(54) **OCCLUSIONSINSTRUMENT UND VERFAHREN ZU DESSEN HERSTELLUNG**
OCCLUSION DEVICE AND METHOD FOR PRODUCING THE SAME
INSTRUMENT D'OCCLUSION ET SON PROCEDE DE PRODUCTION

(30) Priorität: 22.08.2003 DE 10338702
(43) Veröffentlichungstag der Anmeldung: 03.05.2006
(73) Patentinhaber: Occlutech GmbH, 07745 Jena (DE)
(72) Erfinder: MOSZNER, Robert, 07693 Bad Klosterlausnitz (DE); FIGULLA, Hans, Rainer, 07749 Jena (DE); MOSZNER, Friedrich, 99441 Hohlstedt (DE); OTTMA, Rüdiger, 99441 Grossschwabhausen (DE)
(74) Vertreter: Rupprecht, Kay
(86) Internationale Anmeldenummer: PCT/EP2004/009279
(87) Internationale Veröffentlichungsnummer: WO 2005/020822

(56) Entgegenhaltungen:
- WO-A-95/27448
- WO-A-99/12478
- US-A1- 2002 111 647

## Beschreibung

Die vorliegende Erfindung betrifft ein Occlusionsinstrument, welches aus einem Geflecht dünner Drähte oder Fäden besteht, das mittels eines Umformungs- und Wärmebehandlungsverfahrens eine geeignete Form erhält, mit einem proximalen Retentionsbereich, einem distalen Retentionsbereich, wobei in dem distalen Retentionsbereich die Enden der Drähte oder Fäden in einer Fassung zusammenlaufen, und mit einem zylindrischen Steg zwischen dem proximalen und dem distalen Retentionsbereich, wobei die beiden Retentionsbereiche durch einen meist intravaskulären Operationseingriff beiderseits eines zu verschließenden Shunts in einem Septum zur Anlage kommen, während der Steg durch den Shunt hindurchläuft. Die Erfindung betrifft des Weiteren ein Verfahren zur Herstellung dieses Occlusionsinstruments.

In der Medizintechnik besteht seit längerem das Bemühen, septale Defekte, wie etwa Defekte des Vorhofseptums, mittels eines transvenösen, interventionellen Zugangs nichtchirurgisch, also ohne Operation im eigentlichen Sinne, katheterinterventionell zu verschließen. Dabei wurden verschiedene Occlusionssysteme mit unterschiedlichen Vor- und Nachteilen vorgeschlagen, ohne das sich bisher ein bestimmtes Verschluss-System durchsetzen konnte. Im folgenden werden die verschiedenen Systeme "Occluder" oder "Occlusionsinstrumente" genannt. Bei allen interventionellen Occlusionssystemen wird transvenös über einen in einem Septum vorliegenden, zu verschließenden Defekt ein selbst-expandierendes Schirmsystem eingebracht. Ein derartiges System könnte beispielsweise aus zwei Schirmchen bestehen, die jeweils an der distalen Seite (d.h. an der weiter von der Körpermitte bzw. vom Herzen entfernten Seite) bzw. an der proximalen Seite (d.h. an der näher zur Körpermitte angeordneten Seite) des Septums positioniert werden, wobei anschließend die beiden Schirmprothesen im Septum-Defekt zu einem Doppelschirm verschraubt werden. Das Verschluss-System besteht somit dann im zusammengebautem Zustand üblicherweise aus zwei aufgespannten Schirmchen, die über einen kurzen, durch den Defekt hindurchlaufenden Stift miteinander verbunden sind. Bei derartigen aus dem Stand der Technik bekannten Occlusionsinstrumenten stellt es sich jedoch als nachteilig heraus, dass die Implantationsprozedur relativ kompliziert, schwierig und aufwendig ist. Abgesehen von dem komplizierten Implantieren des Verschluss-Systems im zu verschließenden Septum-Defekt besteht bei den verwendeten Schirmchen grundsätzlich die Gefahr der Materialermüdung mit Branchenfraktur. Ferner ist häufig mit thrombembolischen Komplikationen zu rechnen.

Um zu erreichen, dass das erfindungsgemäße Occlusionsinstrument mittels eines Einführbesteckes bzw. eines Führungsdrahtes eingeführt werden kann, ist vorgesehen, dass das Ende des distalen Retentionsbereich eine Fassung aufweist, die in Eingriff mit dem Einführbesteck bzw. Führungsdraht gebracht werden kann. Dabei ist vorgesehen, dass der Eingriff nach der Positionierung des Occlusionsinstruments im Defekt leicht wieder gelöst werden kann. Beispielsweise ist möglich, das Geflecht am Ende des distalen Retentionsbereichs des Occlusionsinstruments derart zu fassen, dass in der Fassung ein Innengewinde hergestellt wird, mit dem das Einführbesteck in Eingriff gelangt. Selbstverständlich sind hier aber auch andere Ausführungsformen denkbar.

Bei einem anderen Occlusionsinstrumenttyp, dem sogenannten Lock-Clamshell-Schirmsystem, sind zwei vorzugsweise mit Dacron bespannte Stahlschirme vorgesehen, die durch je vier Ärmchen stabilisiert werden. Dieser Occludertyp wird über einen venösen Zugang des Patienten implantiert. Bei dem Lock-Clamshell-Occluder hat es sich jedoch als problematisch erwiesen, dass das zur Implantation benötigte Einführbesteck relativ groß ausgeführt werden muss. Ein weiterer Nachteil liegt darin, dass viele verschiedene Occludergrößen benötigt werden, um den jeweiligen Proportionen des zu schließenden Septum-Defekts gerecht zu werden. So hat sich herausgestellt, dass die Schirmchen im eingesetzten Zustand nicht vollständig abflachen, wenn die Länge oder der Durchmesser des im Defekt eingesetzten Stegs nicht optimal passt. Dies führt zu einer unvollständigen Endothelialisierung. Ferner hat es sich gezeigt, dass viele der im Körper des Patienten implantierten Systeme über einen längeren Zeitraum aufgrund der erheblichen mechanischen Belastung Materialermüdungen und Brüche in den metallischen Strukturen aufweisen. Dies ist insbesondere dann der Fall, wenn zwischen dem Implantat und dem Septum dauerhaft Spannungen bestehen.

Um diese Nachteile auszuräumen, wurden selbst-zentrierende Occlusionsinstrumente entwickelt, die mittels minimalinvasiver Verfahren, beispielsweise über einen Katheter und Führungsdrähte, in den Körper des Patienten eingeführt und in den zu verschließenden Septum-Defekt eingebracht werden. Der Konstruktion liegt dabei das Prinzip zugrunde, dass sich das Occlusionsinstrument auf die Größe des für den intravaskulären Operationseingriff verwendeten Einführbesteckes bzw. Katheter verjüngen lässt. Ein derart verjüngtes Occlusionsinstrument wird dann über den Katheter in den zu verschließenden Septum-Defekt bzw. in den zu verschließenden Shunt des Septum-Defektes eingebracht. Danach tritt der Occluder aus dem Katheter aus, worauf sich anschließend die selbst-expandierenden Schirmchen bzw. Retentionsscheibchen entfalten, die sich beiderseits des Septums anlegen. Die Schirme wiederum enthalten beispielsweise aus Dacron gefertigte Gewebeeinlagen oder werden von solchen überspannt, womit der Defekt bzw. Shunt verschlossen wird. Die im Körper verbleibenden Implantate werden nach einigen Wochen bis Monaten mehr oder weniger vollständig von körpereigenem Gewebe eingeschlossen.

Ein Beispiel eines selbst-zentrierenden Occlusionsinstruments der eingangs genannten Art und gemäß dem Oberbegriff des Patentanspruchs 1 ist aus der WO 99/12478 A1 bekannt, das eine Weiterentwicklung des unter dem Namen "Amplatzer-Occluder" bekannten Occlusionsinstruments gemäß der US-Patentschrift Nr. 5,725,552 ist und die Merkmale des Oberbegriffs des Anspruchs 1 aufweist. Er besteht aus einem Geflecht aus einer Vielzahl feiner, geflochtener Nitinol-Drähte in Form eines Jojos. Jenes Geflecht wird in seiner ursprünglichen Form als Rundgeflecht hergestellt, welches sowohl an seinem Anfang (bzw. an seiner proximalen Seite) als auch an seinem Ende (bzw. an seiner distalen Seite) lose Drahtenden aufweist. Bei der Weiterverarbeitung des Rundgeflechtes müssen dann diese losen Enden jeweils in einer Hülse gefasst und verschweißt werden. Nach dieser entsprechenden Weiterverarbeitung weist sowohl die proximale Seite als auch die distale Seite des fertigen Occluders jeweils eine abstehende Hülse auf. In das distale und proximale Retentionsschirmchen und in dem dazwischen angeordneten Steg sind Dacron-Patches eingenäht. Aufgrund des Memory-Effektes des verwendeten Nitinol-Materials entfalten sich die beiden Retentionsschirmchen beim Verlassen des Katheters selbstständig. Dies erfolgt zunächst über eine ballonartige Zwischenstufe, wobei die Retentions-Schirmchen letztendlich beiderseits des Septums entgültig platziert eine mehr oder weniger abgeplattete Form einnehmen. Der Steg zentriert sich während des Aufspannens der Schirmchen in dem zu verschließenden Shunt selbstständig.

Durch diese beim proximalen Retentionsbereich des Occluders hervorstehende Hülse tritt das Problem auf, dass das eingesetzte Implantat emboliebedingte Probleme, insbesondere die konsekutive Embolisation, hervorruft. Dadurch, dass Anteile des Occlusionsinstruments über die Septumwand hervorragen und im ständigen Blutkontakt stehen, werden des weiteren häufig Abwehrreaktion hervorgerufen. Ferner wird oft auch eine vollständige Endothelialisierung des Verschluss-Implantats verhindert.

Des Weiteren ist aus der WO 95/27448 A1 ein aus einem Drahtgeflecht bestehendes Occlusionsinstrument bekannt. Dieses Instrument weist allerdings keine Fassung auf, sodass dieser Occluder auch nicht, wie es bei den oben beschriebenen Instrumenten der Fall ist, auf der selben Weise mittels eines Einführbestecks gesteuert eingebracht oder - im Fall eines falschen Sitzes - noch vor dem Abkoppeln wieder entfernt werden kann.

Der vorliegenden Erfindung liegt von daher die Problemstellung zugrunde, ein solches aus der Medizintechnik bekanntes und aus einem Geflecht bestehendes, selbst-zentrierendes Occlusionsinstrument derart weiterzuentwickeln, dass die vorstehend genannten Nachteile überwunden werden können. Insbesondere soll ein Occlusionsinstrument angegeben werden, das zum Verschluss von Defekten unterschiedlicher Größe anwend-bar ist, wobei die Implantation des Occluders auf einfache Weise erfolgen soll. Ferner soll bei dem Occlusionsinstrument das Auftreten üblicher Komplikationen, wie Dislokation, Teil-Embolisation oder Materialermüdung des Verschluss-Systems, möglichst reduziert werden. Darüber hinaus soll ein Occlusionsinstrument angegeben werden, welches den Verschluss eines Septum-Defektes sicherstellt, wobei möglichst geringe Anteile des Occlusionsinstruments über die Septumwand hervorragen, um so die damit verbundenen und oben genannten Komplikationen zu vermeiden.

Auf der Grundlage dieser Problemstellung liegt der vorliegenden Erfindung die Aufgabe zugrunde, ein Occlusionsinstrument anzugeben, welches im eingesetzten Zustand an der proximalen Seite des Septum-Defektes möglichst flach mit dem Septum abschließt. Der vorliegenden Erfindung liegt ferner das technische Problem zugrunde, ein Verfahren zum Herstellen eines derartigen Occlusionsinstruments anzugeben.

Diese Aufgabe wird bei einem Occlusionsinstrument der eingangs genannten Art erfindungsgemäß dadurch gelöst, dass der proximale Retentionsbereich des Geflechts eine zum proximalen Ende hin offene Form aufweist, wobei die Drähte oder Fäden des geflechts arm offenen Ende des proximalen Retentionsbereiches in Schlingen zum geschlossenen Ende des distalen Retentionsbereiches zuruckgeführt und dort in der Fassung gehalten sind.

Das der vorliegenden Erfindung zugrunde liegende verfahrenstechnische Problem wird ferner durch ein Verfahren zur Herstellung des vorstehend genannten Occlusionsinstruments gelöst, das gekennzeichnet ist durch den Verfahrensschritt des Ausbildens eines trichterförmigen Hohlgeflechts, das an einem ersten, distalen Ende gebündelt und an einem gegenüberliegenden zweiten, proximalen Ende offen ist, und dass gekennzeichnet ist durch den Verfahrensschritt des Ausformens eines proximalen Retentionsbereichs am offenen zweiten Ende, eines distalen Retentionsbereichs am gebündelten ersten Ende und eines zwischen dem proximalen und dem distalen Retentionsbereich angeordneten zylindrischen Stegs.

Die Vorteile der Erfindung liegen insbesondere darin, dass ein intravaskuläres Occlusionsinstrument, insbesondere zur Behandlung von Septum-Defekten, angegeben wird, wobei sich das Verschlussinstrument für eine Zufuhr über einen Katheter zu dem zu verschließenden Defekt eignet. Dadurch, dass der proximale Retentionsbereich des Geflechts eine zum proximalen Ende des Occlusionsinstruments hin offene Form aufweist, kann in besonders vorteilhafter Weise erreicht werden, dass sich das Occlusionsinstrument - unabhängig von der Proportion des Durchmessers des zu verschließenden Defekts und unabhängig von der Stärke der Septumwand - an den Defekt in der Septumwand selbstständig anpasst, und zwar derart, dass an der proximalen Seite des Defekts keine Anteile des Occlusionsinstruments über die Ebene, in welcher die Septumwand mit dem Defekt liegt, herausragt. Somit treten die üblichen hiermit im Zusammenhang stehenden Komplikationen nicht mehr auf. Anders ausgedrückt bedeutet dies, dass das eingesetzte Occlusionsinstrument wesentlich schneller als bei den aus dem Stand der Technik bekannten Verschluss-Systemen vollständig von körpereigenem Gewebe eingeschlossen wird. Aus der Verwendung eines aus dünnen Drähten oder Fäden aufgebauten Geflechts als Ausgangsmaterial für das erfindungsgemäße Occlusionsinstrument leitet sich der weitere Vorteil ab, dass es eine langfristige mechanische Stabilität aufweist. Somit kann das Auftreten von Brüchen in der Struktur des eingesetzten Implantats weitgehend verhindert werden. Ferner besitzt das Geflecht eine ausreichende Steifigkeit. Die zum proximalen Ende hin offene Formgebung des proximalen Retentionsbereiches des Geflechts gestattet es zusätzlich, dass der proximale Retentionsbereich des Instruments im eingesetzten Zustand an dem Randsaum des Defektes vollständig abflacht, und zwar nahezu unabhängig von dem Durchmesser des Defektes und der Stärke der Septumwand. Demnach kann das Occlusionsinstrument über einen weiten Bereich unterschiedlich großer Septum-Defekte eingesetzt werden. Dadurch, dass am proximalen Retentionsbereich auf eine Fassung zum Zusammenbündeln bzw. Zusammenfassen des Geflechtes verzichtet werden kann, ragt auch keine Komponente des Occlusionsinstruments über die Septumwand hinaus, so dass ein ständiger Blutkontakt mit Komponenten des Implantats verhindert werden kann. Dies hat den Vorteil, dass Abwehrreaktionen des Körpers und keine thrombembolischen Komplikationen zu befürchten sind.

Mit dem erfindungsgemäßen Verfahren wird eine besonders leicht zu realisierende Möglichkeit zur Herstellung des vorstehend beschriebenen Occlusionsinstruments angegeben. Dabei wird zunächst ein trichterförmiges Hohlgeflecht mittels beispielsweise einer Rundflechtmaschine ausgebildet. Es kommt hier eine Technik zum Einsatz, bei der das ausgebildete Geflecht am Ende der Flechtlänge, d.h. am späteren distalen Ende des Occlusionsinstruments, gebündelt wird und am Anfang der Flechtlänge, d.h. am späteren proximalen Ende des Occlusionsinstruments, offen bleibt. Damit ist es möglich, ein trichterförmiges Hohlgeflecht herzustellen, dessen gebündeltes Ende dem distalen Ende des fertigen Occlusionsinstruments und dessen gegenüberliegendes offenes Ende dem proximalen Ende des fertigen Occlusionsinstruments entspricht. Dadurch, dass zum Herstellen des Occlusionsinstruments ein an sich bekanntes Flechtverfahren verwendet wird, weist das gefertigte Occlusionsinstrument mechanische Eigenschaften hinsichtlich beispielsweise der Dehnung, Stabilität, Festigkeit etc. auf, die individuell an den späteren Einsatz des Occlusionsinstruments angepasst werden können. In vorteilhafter Weise können metallische Drähte, aber auch organische Fäden zu dem Geflecht verarbeitet werden.

Bevorzugte Weiterbildungen der Erfindung sind bezüglich des Occlusionsinstruments in den Unteransprüchen 2 bis 8 und bezüglich des Herstellungsverfahrens in den Unteransprüchen 10 bis 13 angegeben.

So ist für das Occlusionsinstrument bevorzugt vorgesehen, dass das Geflecht aus Nitinol oder aus einem anderen Material mit Formgedächtnis- oder Memory-Effekt besteht. Als andere Materialien könnten beispielsweise Kupfer-Zink-Aluminium-Legierungen, Gold-Cadmium-Legierungen oder auch Legierungen auf einer Eisen-Basis, wie z.B. Eisen-Mangan-Silicium-Legierungen, aber auch Kunststoffe in Frage kommen, die allesamt dadurch gekennzeichnet sind, dass sie über ein extrem hohes Erinnerungsvermögen verfügen. Für das erfindungsgemäße Occlusionsinstrument ist insbesondere vorgesehen, dass das Geflecht aus einem Formgedächtnispolymer ausgebildet ist, welches beispielsweise auf Polyanhydriden als Matrix oder auf Polyhydroxycarbonsäuren basieren. Hierbei handelt es sich um synthetische, abbaubare Materialien, die über einen thermisch induzierten Formgedächtniseffekt verfügen. Denkbar wären aber auch andere Formgedächtnispolymere, wie etwa Blockcopolymere, wie sie beispielsweise im Sonderdruck Angewandte Chemie 2002, 114, Seiten 2138 bis 2162, von A. Lendlein und S. Kelch beschrieben werden. Durch die Verwendung eines derartigen Materials ist es möglich, als Ausgangskörper für das Occlusionsinstrument ein beispielsweise mittels eines Rundflechtverfahrens erzeugtes, trichterförmiges Hohlgeflecht zu verwenden, welches an seinem Anfang offen und an seinem Ende gebündelt ist. Jener Ausgangskörper wird anschließend mittels eines Umformungs- und Wärmebehandlungsverfahrens in die gewünschte Form des Occlusionsinstruments gebracht. Selbstverständlich sind hier aber auch andere Weiterverarbeitungsverfahren denkbar.

Besonders bevorzugt lässt sich das Geflecht, nachdem es mittels des Umformungs- und Wärmebehandlungsverfahrens eine geeignete Form erhalten hat, auf den Durchmesser eines bei dem intravaskulären Operationseingriff verwendeten Katheters verjüngen. Dadurch ist es möglich, das Occlusionsinstrument zum Verschließen des Defektes durch Einführen des Katheters beispielsweise durch eine Vene einzubringen, so dass eine Operation im eigentlichen Sinne nicht mehr notwendig wird. Wenn das Geflecht aus beispielsweise Nitinol oder aus einem anderen Material mit Formgedächtnis- oder Memory-Effekt besteht, handelt es sich bei dem auf den Durchmesser des Katheters verjüngten Occlusionsinstrument um ein "selbst-expandierendes Instrument", welches sich nach dem Austritt aus dem Katheter selbstständig entfaltet, so dass sich die beiden Retentionsbereiche entsprechend an die proximale bzw. distale Defektseite anlegen können. Die Konstruktion des erfindungsgemäßen Occlusionsinstruments aus dem zusammenhängenden Geflecht ermöglicht es ferner, dass mit dem Occlusionsinstrument ein selbst-expandierendes und selbst-positionierendes Verschluss-System vorliegt, bei welchem das Auftreten von dauerhaft mechanischen Spannungen zwischen dem eingesetzten Occlusions-instrument und der Septumwand verhindert werden kann.

Als mögliche Realisierung ist vorgesehen, dass der proximale Retentionsbereich des Geflechts zu einem proximalen Ende hin offene tulpenförmige Formgebung aufweist.

Denkbar wäre ferner, dass der proximale Retentionsbereich eine zum proximalen Ende hin offene glockenförmige Formgebung aufweist. Demnach kann das Occlusionsinstrument zur Behandlung von verschiedenen Defekten, insbesondere des Ventrikelseptum-Defekts (VSD), Voxhofseptum-Defekts (ASD) sowie persistierenden Duktus arteriosus Botalli (PDA), eingesetzt werden, wobei für eine Vielzahl von Defekten unterschiedlicher Größe und Art grundsätzlich eine optimale Formgebung des proximalen Retentionsbereiches gewählt werden kann. Selbstverständlich sind hier aber auch andere Formgebungen denkbar.

Besonders bevorzugt ist vorgesehen, dass das Occlusionsinstrument wenigstens eine im Steg oder im proximalen Retentionsbereich des Occlusionsinstruments angeordnete Gewebeeinlage aufweist. Diese Gewebeeinlage dient dazu, die in dem Steg und in den sich erweiternden Durchmessern des Occlusionsinstruments verbleibenden Zwischenräume nach dem Einsetzen des Instrumentes im Defekt zu verschließen. Die Gewebeeinlage wird beispielsweise am offenen Ende des Geflechts derart befestigt, dass sie wie ein Tuch über die Öffnung gespannt werden kann. Der Vorteil dieser Konstruktion liegt darin, dass sich der Randsaum des proximalen Retentionsbereiches bündig an das Septum anlegt und weniger Fremdmaterial in den Körper des Patienten eingebracht wird. Die Gewebeeinlagen können beispielsweise aus Dacron hergestellt sein. Selbstverständlich sind hier aber auch andere Materialien und andere Positionen der Gewebeeinlage im bzw. am Occlusionsinstrument denkbar.

Hinsichtlich des Verfahrens ist in bevorzugter Weise vorgesehen, dass der Verfahrensschritt des Ausformens der Retentionsbereiche und des Stegs einen Umformungs- und/oder Wärmebehandlungsschritt aufweist. Dieses ist insbesondere dann von Vorteil, wenn das ausgebildete, trichterförmige Hohlgeflecht aus Nitinol oder aus einem anderen Material, insbesondere Polymer, mit Formgedächtnis- oder Memory-Effekt besteht. Für das erfindungsgemäße Occlusionsinstrument ist insbesondere vorgesehen, dass das Geflecht auf einem Formgedächtnispolymer ausgebildet ist, welches beispielsweise auf Polyanhydriden als Matrix oder auf Polyhydroxycarbonsäuren basieren. Hierbei handelt es sich um synthetische, abbaubare Materialien, die über einen thermisch induzierten Formgedächtniseffekt verfügen. Denkbar wären aber auch andere Formgedächtnispolymere, wie etwa Blockcopolymere, wie sie beispielsweise im Sonderdruck Angewandte Chemie 2002, 114, Seiten 2138 bis 2162, von A. Lendlein und S. Kelch beschrieben werden. Derartige Materialen lassen sich auf einfache Weise durch eine Kombination aus Umformungs- und Wärmebehandlungsschritten in eine entsprechende endgültige Form bringen. Ein fertig ausgebildeter Occluder lässt sich dann beispielsweise auf die Größe eines Katheters verjüngen. Nach dem Austritt aus dem Katheter entfaltet sich das Occlusionsinstrument dann selbstständig und nimmt wieder jene Formgebung an, die mittels des Umformungs- und/oder Wärmebehandlungsschritts dem trichterförmigen Hohlgeflecht des Occlusionsinstruments beim Herstellungsverfahren aufgeprägt wurde.

In bevorzugter Weise ist das trichterförmige Hohlgeflecht derart hergestellt, dass dünne Drähte oder Fäden, die das fertige Geflecht begründen, beim Ausbilden des trichterförmigen Hohlgeflechts am proximalen Ende des Geflechts untereinander verflochten werden. Dies stellt eine mögliche und besonders einfach zu realisierende Art und Weise dar, ein Occlusionsinstrument gemäß der vorliegenden Erfindung herzustellen, dessen proximaler Retentionsbereich eine zum proximalen Ende hin offene Form aufweist. Selbstverständlich sind aber auch andere Herstellungsverfahren denkbar.

Im folgenden werden bevorzugte Ausführungsbeispiele des erfindungsgemäßen Occlusionsinstruments sowie eine Rundflechtmaschine zur beispielhaften Erläuterung des erfindungsgemäßen Herstellungsverfahrens des Occlusionsinstruments anhand der Zeichnungen näher erläutert.

Es zeigen:
- Fig. 1(a): eine schematische Seitenansicht einer bevorzugten ersten Ausführungsform eines erfindungsgemäßen Occlusionsinstruments im expandierten Zustand;
- Fig. 1(b): eine perspektivische Ansicht der ersten Ausführungsform des in Fig. 1(a) gezeigten Occlusionsinstruments im expandierten Zustand;
- Fig. 2(a): eine perspektivische Ansicht einer bevorzugten zweiten Ausführungsform eines erfindungsgemäßen Occlusionsinstruments im expandierten Zustand;
- Fig. 2(b): eine Ansicht der in Fig. 2(a) gezeigten zweiten Ausführungsform des Occlusionsinstruments, bei dem nur die Umrisse dargestellt sind;
- Fig. 3(a): eine dreidimensionale Ansicht einer Rundflechtmaschine zum Erläutern des erfindungsgemäßen Herstellungsverfahrens des Occlusionsinstruments;
- Fig. 3(b): eine Draufsicht auf die in der Fig. 3(a) dargestellte Rundflechtmaschine zum Erläutern des erfindungsgemäßen Herstellungsverfahrens des Occlusionsinstruments;
- Fig. 4(a): einen Flechtkopf der in Figur 3 dargestellten Rundflechtmaschine im Detail; und
- Fig. 4(b): ein mittels des in Fig. 4(a) gezeigten Flechtkopfs hergestelltes Geflecht, welches als Ausgangsgerüst für das erfindungsgemäße Occlusionsinstrument dient.

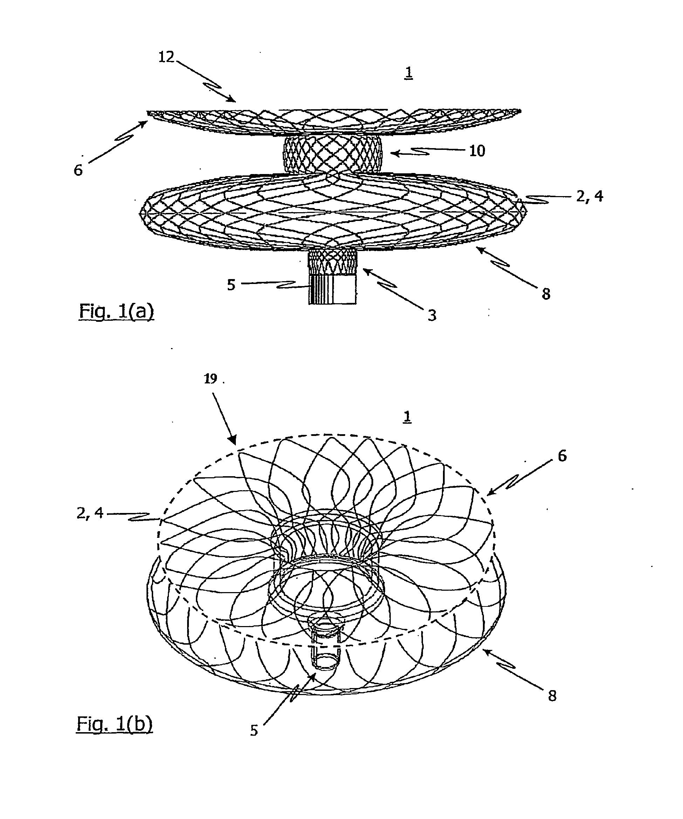

Figur 1(a) zeigt eine schematische Seitenansicht einer bevorzugten ersten Ausführungsform eines erfindungsgemäßen Occlusionsinstruments 1 im expandierten Zustand und Figur 1(b) zeigt eine perspektivische Ansicht der ersten Ausführungsform des in der Figur 1(a) gezeigten Occlusionsinstruments 1. Das Occlusionsinstrument 1 besteht im wesentlichen aus einem Geflecht 2 dünner Drähte oder Fäden 4, die in bevorzugter Weise aus Nitinol oder aus einem anderen Material mit Formgedächtnis- oder Memory-Effekt bestehen. Das Geflecht 2 weist eine ausreichende Flexibilität auf, so dass das Occlusionsinstrument 1 auf den Durchmesser eines bei einem intravaskulären Operationseingriff verwendeten (nicht explizit dargestellten) Katheters verjüngt werden kann. Auf Grund des Memory-Effektes des Materials verfügt das derart verjüngte Occlusionsinstrument 1 eine Formgedächtnisfunktion, so dass sich das Instrument 1 nach dem Verlassen des Katheters selbstständig expandiert und eine der Verwendung entsprechende, vorbestimmte Form wieder einnimmt. Üblicherweise erfolgt dies, nachdem das zunächst im Katheter angeordnete Occlusionsinstrument 1 an der zu behandelnden Stelle platziert wurde.

Wie in den Figuren 1(a) und 1(b) dargestellt, weist das Occlusionsinstrument 1 im expandierten Zustand einen proximalen Retentionsbereich 6, einen distalen Retentionsbereich 8 und einen zylindrischen Steg 10 auf, der zwischen den proximalen und den distalen Retentionsbereich 6, 8 angeordnet ist. Die beiden Retentionsbereiche 6, 8 dienen dazu, einen in einem Septum vorhandenen Defekt bzw. Shunt zu verschließen. Dies erfolgt derart, dass jene Bereiche 6, 8 beiderseits an den zu verschließenden Shunt zur Anlage kommen, während der Steg 10 durch den Shunt hindurchläuft. Das erfindungsgemäße Occlusionsinstrument 1 stellt von daher ein Verschlusssystem dar, das mittels minimalinvasiver Verfahren, d.h. beispielsweise über einen Katheter und Führungsdrähte, dem Körper eines Patienten zugeführt und an der dafür bestimmten Stelle positioniert wird.

Der Konstruktion des erfindungsgemäßen Instruments 1 liegt dabei das Prinzip zu Grunde, das sich das Occlusionsinstrument 1 auf die Größe des Katheters verjüngen lässt. Nach dem Austritt aus dem Katheter entfalten sich dann die Retentionsbereiche 6, 8 selbstständig und legen sich beiderseits des Septums an. Durch die erfindungsgemäße Konstruktion handelt es sich bei dem Occlusionsinstrument 1 ferner um ein selbst-positionierendes und selbst-zentrierendes System. Der Steg 10 hat dabei die Länge der atrialen Scheidewand bzw. des Septums, um eine feste Anlage der Retentionsbereiche 6, 8 an der Septumwand zu gewährleisten.

Im Gegensatz zu den herkömmlichen, aus dem Stand der Technik bekannten Verschlusssystemen, bei denen als proximaler Retentionsbereich 6 ein selbst-expandierendes Schirmchen dient, liegt bei der vorliegenden Erfindung als proximaler Retentionsbereich 6 eine zum proximalen Ende 12 hin geöffnete Glocke bzw. Tulpe vor, so dass keinerlei Material des implantierten Occlusionsinstruments 1 über die Septumwand in den proximalen Bereich des Organs des Patienten hineinragen kann. Durch die zum proximalen Ende 12 hin geöffnete Formgebung des proximalen Retentionsbereiches 6 kann ferner erreicht werden, dass der Rand des proximalen Retentionsbereiches 6 immer bündig mit der Septumwand abschließt. Dies erfolgt über einen relativ weiten Bereich unabhängig von dem Defektdurchmesser und der Stärke der atrialen Scheidewand bzw. des Septums. Dadurch kann erreicht werden, dass nach dem Implantieren des Occlusionsinstrument 1 relativ schnell eine vollständige Endothelialisierung eintritt und mögliche Abwehrreaktionen vom Körper des Patienten nicht eintreten, da ein Blutkontakt mit dem Werkstoff des Implantats 1 wirksam verhindert wird.

Durch die selbstexpandierende Eigenschaft des Implantats 1 aufgrund des Memory-Effektes des verwendeten Materials weist das erfindungsgemäße Occlusionsinstrument 1 eine selbst-zentrierende Funktion im Shunt bzw. im Defekt des Septums auf. Ferner ist das Occlusionsinstrument 1 bis zum Abkoppeln des Führungsdrahtes des Einführbesteckes jederzeit zurückziehbar.

Das erfindungsgemäße Occlusionsinstrument 1 enthält des weiteren Gewebeeinlagen, die in der vorliegenden Zeichnung nicht explizit gezeigt werden. Jene Gewebeeinlagen bestehen meistens aus dem Material Dacron. Denkbar ist dabei, die Gewebeeinlagen im Inneren des Steges 10 oder am proximalen Ende 12 des Retentionsbereiches 6 einzuarbeiten, um den Defekt bzw. Shunt in der Septumwand vollständig verschließen zu können. Die Einlagerung der Gewebeeinlagen kann zum Beispiel durch Verspannung dieser im Occlusionsinstrument 1 erfolgen. Das im Körper eingesetzte Implantat 1 wird dann nach einigen Wochen bzw. Monaten vollständig von körpereigenem Gewebe eingeschlossen.

Das als Grundgerüst für das erfindungsgemäße Occlusionsinstrument 1 dienende Geflecht 2 weist eine ausreichende Steifigkeit auf, dass es die Gewebeeinlage aufspannt und in seiner Position verbleibt.

Am distalen Ende 3 des distalen Retentionsbereiches 8 ist das Geflecht 2 in einer Fassung 5 zusammengefasst. Dies erfolgt dabei derart, dass in der Fassung 5 ein Innengewinde hergestellt werden kann, was dazu dient, mit einem Führungsdraht eines nicht dargestellten Einführbesteckes in Eingriff zu stehen, während das Occlusionsinstrument 1 durch beispielsweise einen intravaskulären Operationseingriff an die entsprechende Position, wo der Defekt in dem Septum vorliegt, gebracht wird. Nach der Positionierung des Occlusionsinstruments 1 im Shunt bzw. Defekt wird der Eingriff zwischen dem Führungsdraht des Einführbesteckes und dem distalen Ende 3 wieder gelöst. Selbstverständlich ist aber auch denkbar, anstelle eines Innengewindes in der Fassung 5 des distalen Ende 3 eine anders ausgeführte Vorrichtung anzubringen.

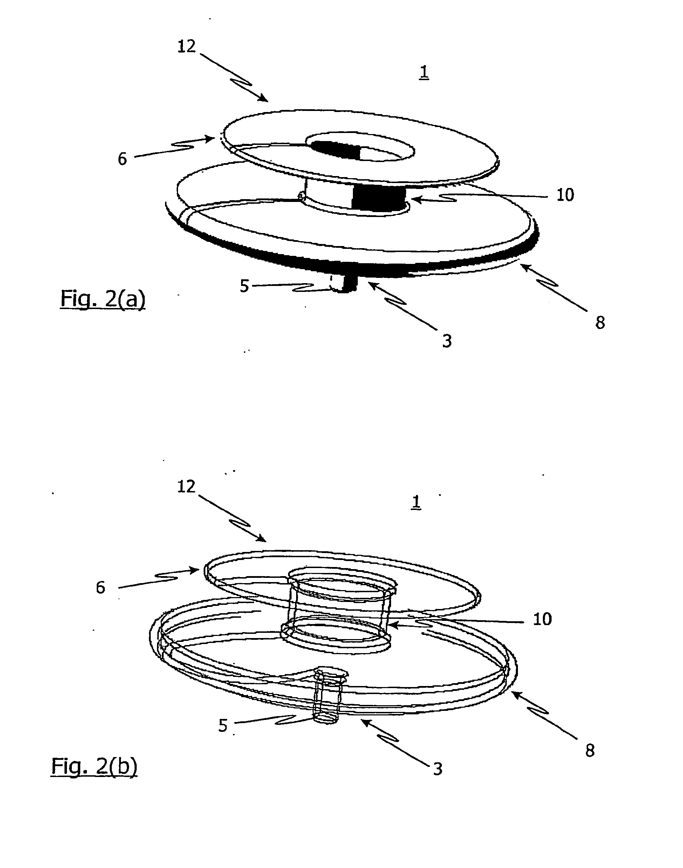

Figur 2(a) ist eine schematische dreidimensionale Ansicht einer bevorzugten zweiten Ausführungsform eines erfindungsgemäßen Occlusionsinstruments 1 im expandierten Zustand und Figur 2(b) ist eine Ansicht der in der Figur 2(a) gezeigten zweiten Ausführungsform des Occlusionsinstruments 1, bei dem zur Vereinfachung nur die Umrisse dargestellt sind. Hier wird zu einer weiteren Vereinfachung auf eine detaillierte Darstellung des als Grundkörper dienenden Geflechtes 2 verzichtet, und die Formgebung des Occlusionsinstruments 1 ist in der Gestalt von geschlossenen Flächen dargestellt. Dieses Occlusionsinstrument 1 weist einen im Vergleich zur ersten Ausführungsform stärker abgeflachten proximalen Retentionsbereich 6 auf. Je nach Verwendungszweck wird der proximale Retentionsbereich 6 mehr oder weniger stark tulpenförmig ausgebildet. Denkbar wären hier aber auch eine zum proximalen Ende 12 hin offene Glockenformen oder eine nahezu tellerförmige Formgebung.

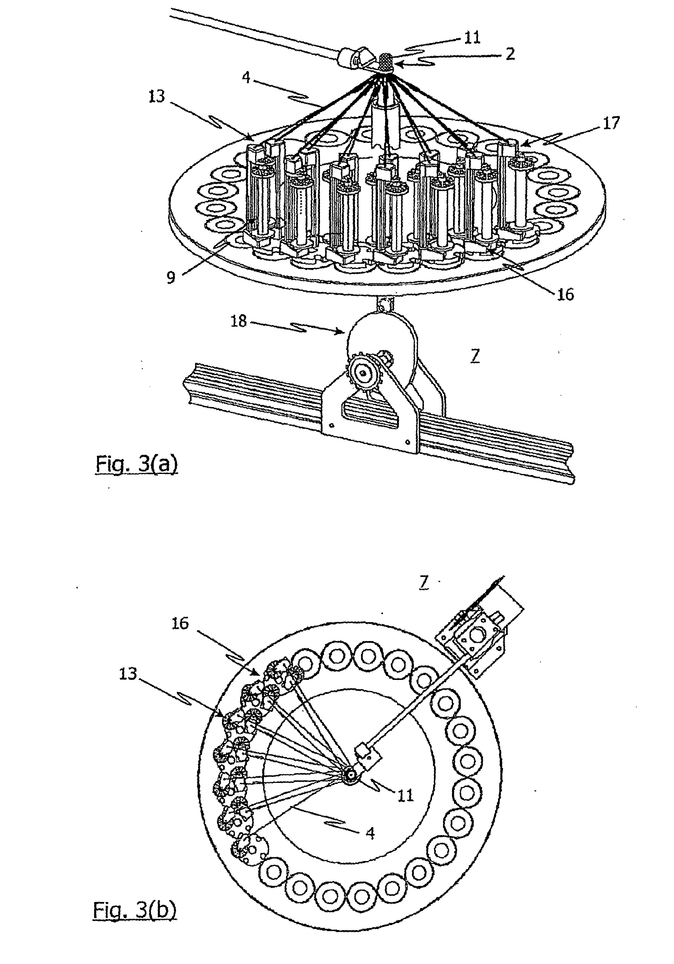

Figur 3(a) zeigt eine Rundflechtmaschine 7 zum Erläutern des erfindungsgemäßen Herstellungsverfahrens des Occlusionsinstruments 1, und Figur 3(b) stellt eine Draufsicht auf die in der Figur 3(a) dargestellte Rundflechtmaschine 7 dar. Im Gegensatz zu den bekannten Flechtverfahren, wo am Anfang des Geflechts 2 alle Fäden oder Drähte 4 in einem Bündel gefasst und zu einer Abzugsvorrichtung hin gespannt werden, wird bei dem erfindungsgemäßen Verfahren der Materialvorrat von einer jeden zweiten Spule 9 zu einem Flechtkopf 11 hin und von diesem zur jeweils einer nächsten Spule 13 oder einem vielfachen Teilungsabstand von derselben hin gespannt. Auf den Spulen 13 ohne Materialvorrat befindet sich nur ein Hilfsfaden, der zumindest bis zum Flechtkopf 11 hinreicht. Das Ende des Materialvorrats wird mit einem Schloss 14 am Ende des Hilfsfadens so weit wie möglich in der Nähe der Spule des Hilfsfadens verbunden.

Der Flechtkopf 11, der in der Figur detailliert dargestellt wird, weist eine kronenförmige Gestalt auf und ist mit Formelementen 15 versehen, die das Einhängen der Fäden bzw. Drähte 4 ermöglichen. Die Formelemente 15 lassen sich versenken, um das Geflecht 2 aushängen und abstreifen zu können. Der Flechtkopf 11 ist im Zentrum der Umlaufbahn von Flügelrädern 16 in einer axialen Anordnung derart platziert, dass die Fäden bzw. Drähte 4 in einem flachen Winkel nach unten zu Klöppeln 17 der Flechtmaschine 7 gerichtet sind.

Nachdem alle für das Geflecht 2 erforderlichen Drähte 4 verbunden und gestrafft sind, beginnt das Flechten in der bekannten Art und Weise, indem die Flügelräder 16 um das Zentrum rotieren, während die Klöppel 17 von Flügelrad zu Flügelrad wechseln und sich dabei gegenseitig auf Ihren Bahnen kreuzen. Der Vorschub für das Geflecht 2 wird über eine Kurvenscheibe 18 in Abhängigkeit der Umläufe der Flügelräder 16 realisiert. Die Geflechtlänge, die mit diesem Verfahren hergestellt werden kann, ist proportional vom Umfang und Steigung des Geflechts 2 sowie von der Länge des Draht- oder Faden-Endes abhängig, welches mit dem Hilfsfaden verbunden ist. Nach dem Flechten werden die freien Enden gebündelt bzw. gefasst, vom Materialvorrat gekappt sowie vom Hilfsfaden entkoppelt. Das so entstandene trichterförmige Hohlgeflecht 2 ist am Anfang offen und am Ende gebündelt. Das Drahtbündel wird so gefasst, dass darin ein Innengewinde hergestellt werden kann, dass mit dem Führungsdraht eines Einführbesteckes in Eingriff bringbar ist.

In dem sich dann anschließenden, werkstoffabhängigen Unformungs- und Wärmebehandelungsverfahren wird das Geflecht 2 in die gewünschte Form des Occlusionsinstruments 1 gebracht. Die Ausgangsform eignet sich zur Herstellung von Occlusionsinstrumenten 1 zur Behandelung eines Ventrikelsektum-Defekts (VSD), Vorhofseptum-Defekts (ASD) sowie persistierenden Duktus arteriosus Botalli (PDA).

Je nach Ausführung werden, von der Fassung 5 aus betrachtet, ein erweiterter Durchmesser (d.h. der distale Retentionsbereich 8) ausgebildet, dem der Steg 10 folgt, an dem sich wieder ein erweiterter, tulpenförmiger Durchmesser (d.h. der proximale Retentionsbereich 6) anschließt. Eine andere Ausführung weist eine oben offene Glockenform auf.

Da das als Grundlage für das Occlusionsinstrument 1 dienende Geflecht 2 als solches einen Defekt nicht vollständig verschließen kann, werden Gewebeeinlagen in den Steg 10 und in den sich erweiternden Durchmessern - den distalen und/oder proximalen Retentionsbereichen 6, 8 - eingebracht. Jene in bevorzugter Weise aus Dacron bestehende Gewebeeinlagen verschließen dann im eingesetzten Zustand des Occlusionsinstruments 1 die im Geflecht 2 verbleibenden Zwischenräume. Die Gewebeeinlage wird beispielsweise so befestigt, dass sie wie ein Tuch über die proximale Öffnung gespannt werden kann.

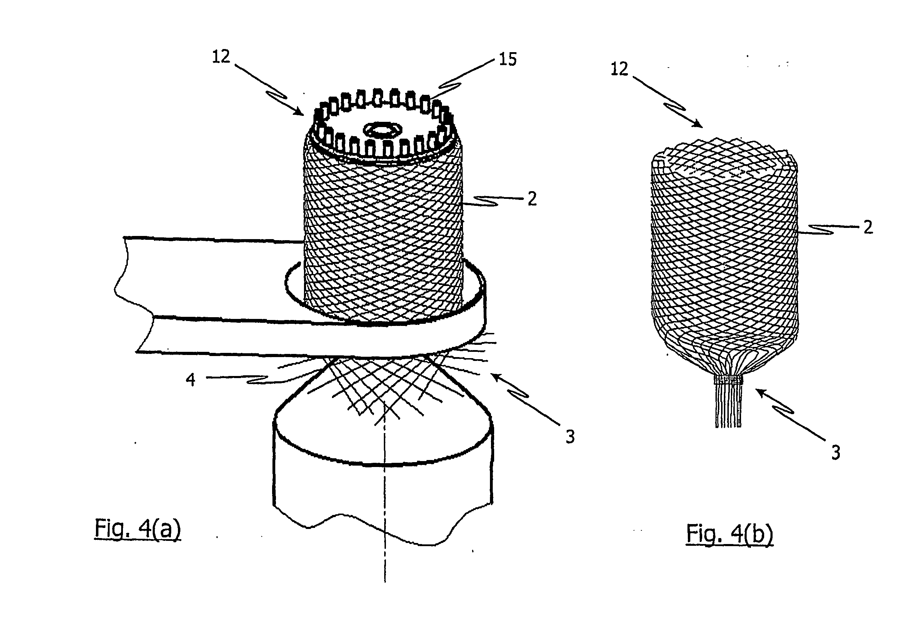

Figur 4(a) zeigt den Flechtkopf 11 der in Figur 3 dargestellte Rundflechtmaschine 7 im Detail und Figur 4(b) zeigt ein mittels des in Figur 4(a) gezeigten Flechtkopfs 11 hergestelltes Geflecht 2, welches als Ausgangsgerüst für das erfindungsgemäße Occlusionsinstrument 1 dient. Hier ist deutlich zu sehen, dass das dem Occlusionsinstrument 1 als Grundkörper dienende Geflecht 2 in Form eines nach oben offenen röhrenförmigen Geflechts 2 gestaltet wird, welches nur an einem Ende 3 mit einer Fassung 5 versehen werden muss, während von der gegenüberliegenden Seite 12 die Fäden bzw. Drähte 4 praktisch aus der Mitte heraus untereinander verflochten werden.

### Bezugszeichenliste

- 1: Occlusionsinstrument
- 2: Geflecht
- 3: distales Ende
- 4: Faden, Draht
- 5: Fassung
- 6: proximaler Retentionsbereich
- 7: Flechtmaschine
- 8: distaler Retentionsbereich
- 9: Spule
- 10: Steg
- 11: Flechtkopf
- 12: proximales Ende
- 13: Spule
- 14: Schloss
- 15: Formelement
- 16: Flügelrad
- 17: Klöppel
- 18: Kurvenscheibe
- 19: Äußerer Rand

## Patentansprüche

1. Occlusionsinstrument, bestehend aus einer Fassung (5) und einem Geflecht (2) dünner Drähte oder Fäden (4), welches mittels eines Umformungs- und Wärmebehandlungsverfahrens eine geeignete Form erhält, mit einem proximalen Retentionsbereich (6), einem distalen Retentionsbereich (8), wobei in dem distalen Retentionsbereich (8) die Enden der Drähte oder Fäden (4) in der Fassung (5) zusammenlaufen, und mit einem zylindrischen Steg (10) zwischen dem proximalen und dem distalen Retentionsbereich (6, 8), wobei die beiden Retentionsbereiche (6, 8) durch einen meist intravaskulären Operationseingriff beiderseits eines zu verschließenden Shunts in einem Septum zur Anlage kommen, während der Steg (10) durch den Shunt hindurchläuft, wobei der proximale Retentionsbereich (6) des Geflechts (2) eine zum proximalen Ende (12) hin offene Form aufweist
**dadurch gekennzeichnet, dass**
die Drähte oder Fäden (4) des Geflechts (2) am offenen Ende (12) des proximalen Retentionsbereiches (6) in Schlingen zum geschlossenen Ende (3) des distalen Retentionsbereiches (8) zurückgeführt und dort in der Fassung (5) gehalten sind.

2. Occlusionsinstrument gemäß Anspruch 1,
**dadurch gekennzeichnet, dass**
das Geflecht (2) aus Nitinol oder aus einem anderen Material mit einem Formgedächtnis- oder Memory-Effekt besteht.

3. Occlusionsinstrument gemäß Anspruch 2,
**dadurch gekennzeichnet, dass**
das Geflecht (2) aus einem Formgedächtnispolymer, insbesondere basierend auf Polyanhydriden als Matrix oder Polyhydroxycarbonsäuren, gebildet ist.

4. Occlusionsinstrument gemäß Anspruch 2
**dadurch gekennzeichnet, dass**
das Geflecht (w) aus Formgedächtnispolymeren in Form von Blockcopolymeren gebildet ist.

5. Occlusionsinstrument gemäß einem der vorstehenden Ansprüche,
**dadurch gekennzeichnet, dass**
sich das Geflecht (2) auf den Durchmesser eines bei dem intravaskulären Operationseingriff verwendeten Katheters verjüngen lässt.

6. Occlusionsinstrument gemäß einem der vorhergehenden Ansprüche,
**dadurch gekennzeichnet, dass**
der proximale Retentionsbereich (6) des Geflechts (2) eine zum proximalen Ende (12) hin offene, abgeflachte tulpenförmige Formgebung aufweist.

7. Occlusionsinstrument gemäß einem der Ansprüche 1 bis 5,
**dadurch gekennzeichnet, dass**
der proximale Retentionsbereich (6) des Geflechts (2) eine zum proximalen Ende (12) hin offene glockenförmige Formgebung aufweist.

8. Occlusionsinstrument gemäß einem der vorhergehenden Ansprüche,
**gekennzeichnet durch**
zumindest eine Gewebeeinlage, welche zum vollständigen Verschließen des Shunts im Steg (10) oder im proximalen Retentionsbereich (6) angeordnet ist.

9. Verfahren zum Herstellen eines Occlusionsinstruments gemäß einem der Ansprüche 1 bis 8,
**gekennzeichnet durch**
folgende Verfahrensschritte:
a) Ausbilden eines trichterförmigen Hohlgeflechts (2) mittels eines an sich bekannten Flechtverfahrens, wobei das Hohlgeflecht (2) an einem ersten, distalen Ende (3) gebündelt wird und an einem gegenüberliegenden zweiten, proximalen Ende (12) offen bleibt; und
b) Ausformen eines proximalen Retentionsbereichs (6) am offenen zweiten Ende (12), eines distalen Retentionsbereichs (8) am gebündelten ersten Ende (3) und eines zwischen dem proximalen und dem distalen Retentionsbereich (6, 8) angeordneten zylindrischen Stegs (10).

10. Verfahren gemäß Anspruch 9,
**gekennzeichnet durch**
den Verfahrensschritt des Ausbildens einer Fassung (16) am gebündelten distalen Ende (3) des trichterförmigen Hohlgeflechts (2).

11. Verfahren gemäß Anspruch 9 oder 10,
**dadurch gekennzeichnet, dass**
die Drähte und Fäden (4) des Geflechts (2) am äußeren Rand (19) der abgeflachten Tulpenform des offenen Endes (12) des proximalen Retentionsbereiches (6) in Schlingen zum geschlossenen Ende (3) des distalen Retentionsbereiches (8) zurückgeführt werden und dort in der Fassung (16) gebündelt und befestigt werden.

12. Verfahren gemäß Anspruch 9 oder 10,
**dadurch gekennzeichnet, dass**
der Verfahrensschritt des Ausformens der Retentionsbereiche (6, 8) und des Stegs (10) eine Umformungs- und/oder Wärmebehandlung enthält.

13. Verfahren nach einem der Ansprüche 9 bis 12,
**dadurch gekennzeichnet, dass**
das trichterförmige Hohlgeflecht (2) derart hergestellt wird, dass dünne Drähte oder Fäden (4), die das fertige Geflecht (2) ausbilden, beim Ausbilden des trichterförmigen Hohlgeflechts (2) am proximalen, offenen Ende (12) des Geflechts (2) untereinander verflochten werden.

## Claims

1. Occlusion device, consisting of a frame (5) and a weave (2) of thin wires or threads (4), which is suitably shaped by means of shaping and heat treatment processes, with a proximal retention zone (6), a distal retention zone (8), the ends of the wires or threads (4) converging together in the frame (5) in the distal retention zone (8), and a cylindrical waist (10) between the proximal and distal retention zone (6,8), both retention zones (6,8) being arranged on either side of a shunt to be closed in a septum usually by intravascular operation, while the waist (10) runs through the shunt, the proximal retention zone (6) of the weave (2) opening towards the proximal end (12)
**characterised in that**
the wires or threads (4) of the weave (2) at the open end (12) of the proximal retention zone (6) are connected in loops to the closed end (3) of the distal retention zone (8) and are held there in the frame (5).

2. Occlusion device according to claim 1,
**characterised in that**
the weave (2) consists of Nitinol or another material with a shape memory effect or memory effect.

3. Occlusion device according to claim 2,
**characterised in that**
the weave (2) is formed from a shape memory polymer, particularly based on polyanhydrides as matrix or polyhydroxy carbonic acids.

4. Occlusion device according to claim 2
**characterised in that**
the weave (2) is formed from shape memory polymers in the shape of block copolymers.

5. Occlusion device according to any of the preceding claims
**characterised in that**
the weave (2) can be contracted to the diameter of the catheter used in the intravascular operation.

6. Occlusion device according to any of the preceding claims,
**characterised in that**
the proximal retention zone (6) of the weave (2) displays a flattened-edge tulip shape open towards to the proximal end (12).

7. Occlusion device according to one of claims 1 to 5,
**characterised in that**
the proximal retention zone (6) of the weave (2) displays a bell shape open towards the proximal end (12).

8. Occlusion device according to any of the preceding claims,
**characterised by**
at least one tissue implant, which is arranged in the waist (10) or in the proximal retention zone (6) for the complete closure of the shunt.

9. Procedure to manufacture an occlusion device according to any of claims 1 to 8,
**characterised by**
the following procedural steps:
a) formation of a funnel-shaped hollow weave (2) using a weaving process known *per se,* the hollow weave (2) being drawn together at a first, distal end (3) and remaining open at a second, proximal end (12) located opposite; and
b) shaping a proximal retention zone (6) at the open second end (12), a distal retention zone (8) at the drawn-in first end (3) and a cylindrical waist (10) arranged between the proximal and the distal retention zone (6,8).

10. Procedure according to claim 9,
**characterised by**
the procedural step of forming a frame (16) at the drawn-in distal end (3) of the funnel-shaped hollow weave (2).

11. Procedure according to claim 9 or 10,
**characterised in that**
the wires and threads (4) of the weave (2) at the outer edge (19) of the flattened-edge tulip shape of the open end (12) of the proximal retention zone (6) are looped back to the closed end (3) of the distal retention zone (8) and are drawn together there and secured in the frame (16).

12. Procedure according to claim 9 or 10,
**characterised in that**
the procedural step of shaping the retention zone (6,8) and the waist (10) contains shaping and/or heat treatment.

13. Procedure according to any of claims 9 to 12,
**characterised in that**
the funnel-shaped hollow weave (2) is manufactured in such a way that thin wires or threads (4), that form the finished weave (2), are interwoven at the proximal, open end (12) of the weave (2) to form the funnel-shaped hollow weave (2).

## Revendications

1. Instrument d'occlusion, composé d'une monture (5) et d'une tresse (2) de fils minces ou filaments (4), qui, au moyen d'un procédé de déformation et de traitement thermique, obtient une forme appropriée, comportant une zone de rétention proximale (6), une zone de rétention distale (8), dans lequel, dans la zone de rétention distale (8), les extrémités des fils minces ou filaments (4) pénètrent dans la monture (5), et comportant une tige cylindrique (10) entre la zone de rétention proximale et la zone de rétention distale (6, 8), les deux zones de rétention (6, 8) prenant appui, par le biais d'une intervention chirurgicale le plus souvent intravasculaire, des deux côtés d'un shunt d' un septum à refermer, alors que la tige (10) traverse le shunt, la zone de rétention proximale (6) de la tresse (2) présentant une forme ouverte vers l'extrémité proximale (12),
**caractérisé en ce que**
les fils minces ou filaments (4) de la tresse (2), au niveau de l'extrémité ouverte (12) de la zone de rétention proximale (6), sont ramenés en boucle vers l'extrémité fermée (3) de la zone de rétention distale (8) et y sont maintenus dans la monture (5).

2. Instrument d'occlusion selon la revendication 1, **caractérisé en ce que** la tresse (2) se compose de nitinol ou d'un autre matériau possédant un effet de mémoire ou à mémoire de forme.

3. Instrument d'occlusion selon la revendication 2, **caractérisé en ce que** la tresse (2) est formée d'un polymère à mémoire de forme, en particulier à base de polyanhydrides en tant que matrice ou d'acides polyhydroxycarboxyliques.

4. Instrument d'occlusion selon la revendication 2, **caractérisé en ce que** la tresse (w) est formée de polymères à mémoire de forme sous la forme de polymères séquencés.

5. Instrument d'occlusion selon l'une des revendications précédentes, **caractérisé en ce que** la tresse (2) se rétrécit au diamètre d'un cathéter utilisé lors d'une intervention chirurgicale intravasculaire.

6. Instrument d'occlusion selon l'une des revendications précédentes, **caractérisé en ce que** la zone de rétention proximale (6) de la tresse (2) présente un façonnage en forme de tulipe aplatie, ouverte vers l'extrémité proximale (12).

7. Instrument d'occlusion selon l'une des revendications 1 à 5, **caractérisé en ce que** la zone de rétention proximale (6) de la tresse (2) présente un façonnage en forme de cloche ouverte vers l'extrémité proximale (12).

8. Instrument d'occlusion selon l'une des revendications précédentes, **caractérisé par** au moins un insert de tissu qui est disposé, pour fermer totalement le shunt, dans la tige (10) ou dans la zone de rétention proximale (6).

9. Procédé de fabrication d'un instrument d'occlusion selon l'une des revendications 1 à 8, **caractérisé par** les étapes de procédé suivantes :
a) élaboration d'une tresse creuse en forme d'entonnoir (2) au moyen d'un procédé de tressage connu en soi, dans lequel la tresse creuse (2) est concentrée au niveau d'une première extrémité distale (3) et reste ouverte au niveau d'une seconde extrémité, proximale, opposée (12) ; et
b) façonnage d'une zone de rétention proximale (6) au niveau de la seconde extrémité ouverte (12), d'une zone de rétention distale (8) au niveau de la première extrémité concentrée (3) et d'une tige cylindrique (10) disposée entre les zones de rétention proximale et distale (6, 8).

10. Procédé selon la revendication 9, **caractérisé par** une étape de formation d'une monture (16) au niveau de l'extrémité distale concentrée (3) de la tresse creuse en forme d'entonnoir (2).

11. Procédé selon la revendication 9 ou 10, **caractérisé en ce que** les fils minces et filaments (4) de la tresse (2), au niveau du bord extérieur (19) de la forme de tulipe aplatie de l'extrémité ouverte (12) de la zone de rétention proximale (6), sont ramenés en boucle vers l'extrémité fermée (3) de la zone de rétention distale (8) et y sont concentrés et fixés dans la monture (16).

12. Procédé selon la revendication 9 ou 10, **caractérisé en ce que** l'étape de façonnage des zones de rétention (6, 8) et de la tige (10) contient un traitement de déformation et/ou un traitement thermique.

13. Procédé selon l'une des revendications 9 à 12, **caractérisé en ce que** la tresse creuse en forme d'entonnoir (2) est fabriquée de façon telle que les fils minces ou filaments (4) qui forment la tresse finie (2), lors de l'élaboration de la tresse creuse en forme d'entonnoir (2), sont entrelacés entre eux au niveau de l'extrémité proximale ouverte (12) de la tresse (2).
